# EUROPEAN PATENT APPLICATION

(11) **EP 1 894 629 A2**
(43) Date of publication of application: **05.03.2008**
(21) Application number: 07253282.3
(22) Date of filing: 20.08.2007
(51) Int. Cl.: B01J 47/00, B01J 49/00

(54) **Method and composition for eliminating odors in ion exchange resins**

(30) Priority: 01.09.2006 US 842000 P
(71) Applicant: Rohm and Haas Company, Philadelphia, PA 19106-2399 (US)
(72) Inventor: Hughes, Lyn, Pennsylvania 19438 (US)
(74) Representative: Buckley, Guy Julian

(57) **Abstract**

A method for reducing undesirable odor of an anion exchange resins by preparing a composition comprising an admixture comprising an anion exchange resin having an undesirable odor and a cation exchange resin; and converting the mixture to form the composition.

## Description

The invention relates to methods and compositions for eliminating odor in ion exchange resins. In particular, this invention relates to eliminating odor in anion exchange resins having an undesirable odor.

A common problem associated with anion exchange resins is that they sometimes have a foul odor that consumers find unpleasant. One cause of the odor is believed to be the result of slow degradation of the resin that generates volatile compounds, such as ammonia and amines as by-products. The odor problem is of particular importance in pharmaceuticals, neutraceuticals, and cosmeceuticals. This is exemplified in the pharmaceutical industry by cholestyramine, which is an anion exchange resin used to reduce serum cholesterol in hypercholesterolemic patients. The odor of cholestyramine is described as fishy and is sufficiently objectionable to consumers to reduce compliance with dosing requirements.

Others have addressed the problem of odor by adding components, including flavorings; spices and sweeteners, to the products to mask the smell. For example, WO 2004/045588 discloses a formulation having a flavoring added to mask or disguise or neutralize the odor.

In the present invention, the problem of odor is solved by adding a cation exchange resin to absorb the odor of the anion exchange resin and thus, reduce the foul smell, also referred to herein as an "undesirable odor."

One aspect of this invention provides a composition comprising an anion exchange resin having an undesirable odor and a cation exchange resin, where a ratio of cation exchange resin to anion exchange resin being 0.1:99.9 to 25:75.

Another aspect of this invention provides a process for preparing a composition comprising providing an admixture comprising an anion exchange resin having an undesirable odor and a cation exchange resin, where a ratio of cation exchange resin to anion exchange resin being 0.1:99.9 to 25:75; and converting the mixture to form the composition.

The term "moisture retention capacity" of an ion exchange resin used herein refers to the amount of water retained within the ion exchange resin when in contact with an excess of amount of water. An alternative name for moisture retention capacity is moisture holding capacity.

The term "exchange capacity" of an ion exchange resin used herein refers to the maximum amount of exchangeable ions the ion exchange resin can contain. Thorough descriptions of ion exchange resin technology, including descriptions of water retention capacity, exchange capacity, functional groups, polymer structure, manufacture, and uses, can be found in the open literature in books such as Ion Exchange Resins. 2nd ed. (Robert Kunin, 1972, Robert E. Krieger, Huntington, N. Y.) and Ion Exchange (Fiedrich Helfferich, 1962, McGraw-Hill Book Co., New York).

The invention is useful for anion exchange resins having undesirable odors. The anion exchange resins may be active ingredients or inactive ingredients in formulations. The anion exchange resins may also have at least one active ingredient loaded into it. Active ingredients useful in the practice of this invention are those that include acidic ionizable groups and are pharmaceuticals, neutraceuticals or cosmeceuticals. Exemplary active ingredients include, but are not limited to, acetaminophen, ambroxol, p-aminobenzoic acid, ascorbic acid (vitamin C), biotin (vitamin B7), essential fatty acids (vitamin F), cyclic acid, diclofenac, ellagic acid, glycolic acid, hyaluronic acid, α-hydroxy acids, p-hydroxy acids, ibuprofen, indomethacin, isostrearic acid, isotretinoin, ketoprofen, lactic acid, lansoprazole, linoleic acid, α-lipoic acid, mefanamic acid, naproxen, nelfinavir, nicotinic acid, omeprazole, polyhydroxy acids, progabide, pyritinol, quinapril, repaglinide, riboflavin (vitamin B2), salicylic acid, sorbic acid, stearic acid, sulindac, topiramate, valproic acid, vitamin E, vitamin K₅, and vitamin U.

When the anion exchange resin is loaded with an active ingredient, the active ingredient component of the composition may be present in any amount which is sufficient to elicit a beneficial effect. Preferably, the loading of active ingredient in the resinate of the present invention is 1-100% of the ion exchange capacity of the resin, more preferably, it is 5-95% of the ion exchange capacity of the resin, and most preferably, it is 10-90% of the ion exchange capacity of the resin.

The anion exchange resin may be combined with an active ingredient to form a resinate in which the active ingredient is ionically bound to the anion exchange resin. Such active ingredient needs to be an acidic, ionizable molecule so that its ionized form can interact with the basic groups of the anion exchange resin to form the ionic bond. When resinates are exposed to fluids, such as physiological fluids, the active ingredient can be released from the resinate by the mechanism of ion exchange.

Anion exchange resins are insoluble, crosslinked polymers that contain ionizable basic groups attached to the polymer backbone. They are capable of exchanging anions with solution into which they come in contact. In weakly basic anion exchange resins, the basic groups are primary, secondary, or tertiary amines. In strongly basic anion exchange resins, the basic groups are quaternary amines. Weakly basic anion exchange resins useful in the present invention are in the free base form, salt form, or the partial salt form. Strongly basic anion exchange resins useful in the present invention are in the salt form. Weakly basic anionic exchange resins are preferred.

The anion exchange resin of the invention is a polymer that has an undesirable odor, meaning that it is perceived by an average person to have a foul or unpleasant smell. The undesirable odor may be produced by base compounds, for example, ammonia and organic amines. An organic amine is a compound in which the N atom of an amine group is covalently bonded to at least one and not more than four carbon atoms. Said amine group can be a primary, secondary, tertiary, or quaternary amine. Said carbon atoms can part of any organic molecule, included but not limited to aromatic, aliphatic, carbocyclic, and heterocyclic molecules. Examples include, but are not limited to, methyl amine, dimethyl amine, ethyl amine, dimethyl amine, trimethyl amine, trimethyl amine, pyridine, and aniline. The amine can also be present as a salt of an acid, for example, hydrochlorides.

One such anion exchange resin is cholestyramine resin, which has an amine odor. Other anion exchange resins having an undesirable odor include strongly basic anion exchange resins, such as Amberlite® IRA900 OH and Amberlite® 958 Cl (manufactured by Rohm and Haas Company, Philadelphia, PA) and Dowex Marathon® 550A and Dowex Upcore® Mono MA-600 (manufactured by Dow Chemical, Wilmington, DE), and weakly basic anion exchange resins, such as Amberlite® IRA96 (manufactured by Rohm and Haas Company, Philadelphia, PA) and Dowex® 66 and Dowex Marathon® WBA (manufactured by Dow Chemical, Wilmington, DE).

Cation exchange resins are insoluble, crosslinked polymers that contain ionizable acidic groups attached to the polymer backbone. They are capable of exchanging cations with solutions into which they come in contact. In weakly acidic cation exchange resins, the acidic groups are weakly ionizable acidic groups, such as carboxylic acids and phenols. In strongly strongly acidic cation exchange resins, the acidic groups are strongly ionizable acidic groups, such as sulfonic acids.

Cation exchange resins useful in this invention are strongly acidic cation exchange resins and weakly acidic cation exchange resins. Preferred cation exchange resins are weakly acidic cation exchange resins. More preferred cation exchange resins are weakly acidic cation exchange resins with a carboxylic functionality. Examples of weakly acidic cation exchange resins with carboxylic functionality include Amberlite® IRP64, Amberlite® IRP88, and Amberlite® IRC50 (manufactured by Rohm and Haas Co, Philadelphia, PA).

Cation exchange resins useful in the practice of the present invention are in the acid form or salt form or partial salt form. Cation and anion exchange ("ion") resins useful in this invention have a moisture content between 0% and the moisture retention capacity of said resin.

Ion exchange resins are manufactured in different forms. These forms include spherical and non-spherical particles. The non-spherical particles are frequently manufactured by grinding of the spherical particles. The spherical particles are frequently known in the art as "whole bead." The non-spherical particles are frequently known in the art as "powders."

Anion exchange resin useful in the present invention can be of any shape or size. Cation exchange resins useful in the present invention can be of any shape. The size of cation exchange resins useful in the present invention is, preferably, 0.00001mm to 2mm. The more preferred size is 0.00 1 mm to 1.0mm, and the most preferred size is 0.001 to 0.3mm.

The ratio of cation exchange resin to anion exchange resin useful in the present invention is, preferably, from 0.1:99.9 to 25:75. A more preferred ratio of cation exchange resin to anion exchange resin useful in the present invention is from 0.1:99.9 to 10:90, and a most preferred ratio of cation exchange resin to anion exchange resin useful in the present invention is from 0.2:99.8 to 5:95.

The composition has pharmacological, nutritional and/or cosmetic properties, so the present invention may be used in pharmaceutical, neutraceutical, and cosmeceutical applications. In pharmaceutical and neutraceutical applications, the composition may be used in any type of medium where the active ingredient is delivered to a patient's system. These include, but are not limited to, tablets, capsules, pills, suspensions, troches, creams, ointments, transdermals, and powders. In cosmeceutical applications, the composition may be used in powders, creams, emulsions, ointments, lotions, foams, gels, powders, transdermal and other formulations. In one embodiment, the composition of the invention is administered to patients/consumers orally.

The composition may also contain additives, including, but not limited to, pharmaceutically active ingredients, cosmeceuticals, , neutraceuticals, , flavors, fragrances, glidants, fillers, release-rate modifying agents, sweeteners, and disintegrants. Compositions of the present invention may be coated or uncoated.

### EXAMPLES

### Example 1: Evaluation of the effect of Amberlite® IRP64 on the odor of Duolite® AP143

Amberlite® IRP64 is a resin derived form a porous copolymer of methacrylic acid and divinylbenzene and available from Rohm and Haas Company, Philadelphia, PA. Duolite® AP143 (cholestyramine available from Rohm and Haas Company, Philadelphia, PA) is a strongly basic anion exchange resin in the chloride form. It is a dry, finely ground solid used in pharmaceutical formulations. Several samples of mixtures of AP143 and IRP64 were prepared and evaluated for amine odor within an hour of preparation. The evaluation was done by three different observers (Obs 1, Obs 2, and Obs 3) and ranked on a scale of 0 - 5 as follows:

| | |
|---|---|
| 0 | None |
| 1 | Faint/trace |
| 2 | Definite odor, but weak |
| 3 | Moderate odor |
| 4 | Stronger odor |
| 5 | Strong (defined as untreated cholestyramine) |

The results are given in Table 1.

**Table 1**

| % w/w IRP64 | Obs 1 | Obs 2 | Obs 3 | Mean |
|---|---|---|---|---|
| 0 | 5 | 5 | 5 | 5 |
| 0.3 | 2 | 2 | 3 | 2.3 |
| 1 | 0 | 0 | 3 | 1 |
| 5 | 0 | 0 | 1 | 0.3 |
| 20 | 0 | 0 | 2 | 0.7 |

The results indicate a reduction in amine odor with the addition of IRP64. Increasing the amount of IRP64 reduces the odor.

The samples we re-evaluated by Observer 1 after ~1 month. The odor scores were the same as the 1 hour scores reported in Table 1.

### Example 2: Evaluation of the effect of Amberlite® IRP64 on the odor of Amberlite® IRA900 OH

IRA900 OH is a whole bead strongly basic anion exchange resin in the hydroxide form supplied as a wet solid. It is used in water treatment and is characterized by a strong amine odor due to the high pH of the material. The odor associated with this product is typically rated as stronger than that of AP143.

35ml of wet resin was mixed with 1 g of IRP64, (approximately 4.4% w/w), and the odor was evaluated within 5 minutes by three observers (Observer 1, Observer 4, and Observer 5). In all three cases, the odor was scored as 0.

## Claims

1. A composition comprising:
an anion exchange resin having an undesirable odor; and
a cation exchange resin, a ratio of cation exchange resin to anion exchange resin being 0.1:99.9 to 25:75.

2. The composition of claim 1 wherein the undesirable odor is caused by at least one of an ammonia and an organic amine.

3. The composition of claim 1 wherein the anion exchange resin comprises at least one of a weakly basic anion exchange resin and a strongly basic anion exchange resin.

4. The composition of claim 1 wherein the anion exchange resin comprises cholestyramine.

5. The composition of claim 1 wherein the cation exchange resin comprises a weakly acidic exchange resin having carboxylic functionality.

6. The composition of claim 1 wherein the ratio comprises 0.2:99.8 to 5:95.

7. The composition of claim 1 wherein the anion exchange resin comprises an active ingredient.

8. The composition of claim 1 wherein the composition comprises at least one of a pharmaceutical, a neutraceutical, and a cosmeceutical composition.

9. A process for preparing a composition comprising
providing an admixture comprising an anion exchange resin having an undesirable odor and a cation exchange resin, a ratio of cation exchange resin to anion exchange resin being 0.1:99.9 to 25:75; and
converting the mixture to form the composition.

10. The process of claim 9 wherein the anion exchange resin comprises at least one of a weakly basic anion exchange resin and a strongly basic anion exchange resin and the acidic cation exchange resin comprises a weakly acidic exchange resin having carboxylic functionality.
